# EUROPEAN PATENT APPLICATION

(11) **EP 1 642 900 A1**
(43) Date of publication of application: **05.04.2006**
(21) Application number: 04746198.3
(22) Date of filing: 21.06.2004
(51) Int. Cl.: C07D 487/04, A61K 31/55, A61P 37/08

(54) **TRICYCLIC TRIAZOLOBENZAZEPINE DERIVATIVE PRODUCED AS NOVEL CRYSTALLINE SUBSTANCE**

(30) Priority: 19.06.2003 JP 2003175347
(71) Applicant: Meiji Seika Kaisha, Ltd., Tokyo 104-8002 (JP)
(72) Inventor: KITAHARA, Shinichi, c/o Meiji Seika Kaisha, Ltc., Yokohama-shi, Kanagawa 2228567 (JP); YAMAGUCHI, Toshihiro, c/o Meiji Seika Kaisha, Ltc., Yokohama-shi, Kanagawa 2228567 (JP)
(74) Representative: Ahner, Francis
(86) International application number: PCT/JP2004/008729
(87) International publication number: WO 2004/113343

(57) **Abstract**

The present invention provides a novel crystalline compound of the tricyclic triazolobenzazepine derivative, excellent in solubility and absorbability.

## Description

### BACKGROUND OF THE INVENTION

### Field of the Invention

The present invention relates to a novel crystalline material of 2-(1-isopropoxycarbonyloxy-2-methyl propyl)-7 ,8-dimethoxy-4(5H),10-dioxo-2H-1,2,3-triazolo[4,5-c][1]benzazepi ne, useful for pharmaceuticals.

### Background Art

2-(1-Isopropoxycarbonyloxy-2-methylpropyl)-7,8-dimeth oxy-4(5H),10-dioxo-2H-1,2,3-triazolo[4,5-c][1]benzazepine (hereinafter referred to as "Compound A") is a compound representded by the following chemical formula, which is expected to use as an antiallergic agent, as is described in WO 99/16770 (Japanese Patent Publication No. 3,188,482 and US Patent No. 6,372,735) (the descriptions in these patent publications are herein incorporated by reference).

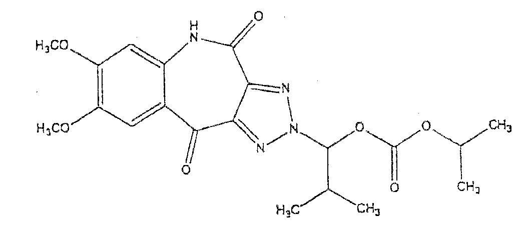

A stable crystalline compound of Compound A (hereinafter referred to as the "α-type crystalline material") can be obtained by producing crude Compound A in accordance with the process described in the aforementioned patent publications, dissolving it in methylene chloride and recrystallizing the material with methanol. However, it became evident from our experiments that the α-type crystalline material is sparingly soluble in solvents such as water and that it cannot be easily absorbed into organisms when administered as it is. To design and produce preparations that can exhibit the advantageous pharmacological activity of Compound A, it is desirable to develope a novel physical form of Compound A, which is excellent in both solubility and absorbability.

We have now succeeded in obtaining a novel crystalline material of Compound A, excellent in solubility and absorbability. The present invention is based on this finding.
An object of the present invention is, therefore, to provide a novel crystalline material of Compound A, excellent in both solubility and absorbability.

### SUMMARY OF THE INVENTION

Crystalline compound of Compound A according to the present invention, which has the diffraction peaks at the diffraction angles (2θ) of: 4.7 ± 0.1°, 7.4 ± 0.1°, 11.8 ± 0.1°, 13.4 ± 0.1°, 16.5 ± 0.1°, and 18.6 ± 0.1° in a powder X-ray diffraction pattern.

The crystalline compound of Compound A according to the present invention can show high solubility in water and in aqueous organic solvents such as methanol and ethanol. Moreover, the crystalline compound of Compound A according to the invention is readily absorbed into organisms and has significantly high bioavailability.

### BRIEF DESCRIPTION OF THE DRAWINGS

Fig. 1 is a powder X-ray diffraction pattern of the crystalline compound of Compound A obtained in Example 1,
Fig. 2 is a powder X-ray diffraction pattern of the crystalline compound of Compound A obtained in Comparative Example 1,
Fig. 3 is a chart showing a DSC curve of the crystalline compound of Compound A obtained in Example 1,
Fig. 4 is a chart showing a DSC curve of the crystalline compound of Compound A obtained in Comparative Example 1,
Fig. 5 is a diagram showing the warm water solubility of the crystalline compound of Compound A obtained in Example 1 and that of the crystalline compound of Compound A obtained in Comparative Example 1, and
Fig. 6 is a diagram showing the concentration change of the Compound B in blood plasma in the case where the crystalline compound of Compound A obtained in Example 1 or that obtained in Comparative Example 1 was suspended in a 1 wt.% aqueous solution of methyl cellulose and the suspension was orally administered to cynomolgus monkeys.

### DETAILED DESCRIPTION OF THE INVENTION

The crystalline compound of Compound A according to the present invention has the diffraction peaks at the diffraction angle (2θ): 4.7 ± 0.1°, 7.4 ± 0.1°, 11.8 ± 0.1°, 13.4 ± 0.1°, 16.5 ± 0.1°, and 18.6 ± 0.1° in a powder X-ray diffraction pattern. Further, this crystalline compound has a broad endothermic peak approximately at 170 - 190°C and a sharp endothermic peak approximately at 225°C in a DSC chart obtained in differential scanning calorimetry (DSC). As far as we know, a Compound A product having such specific, identified physicochemical properties has not yet been known. It can, therefore, be said that this crystalline compound of Compound A is novel.

Furthermore, the crystalline compound of Compound A according to the present invention can be used for the prophylaxis or treatment of allergic diseases. Allergic diseases herein include bronchial asthma, eczema, urticaria, allergic gastrointestinal disorders, allergic rhinitis, and allergic conjunctivitis. Therefore, according to another aspect of the present invention, there is provided a composition, particularly a pharmaceutical composition, comprising the crystalline compound of Compound A according to the invention.

Although the crystalline compound of Compound A according to the present invention may be orally administered as it is, it can usually be formulated, together with known pharmaceutically acceptable carriers, into compositions.
For oral administration, the crystalline compound of Compound A according to the present invention may be formulated, together with known pharmaceutically acceptable excipients (e.g., lactose, crystalline cellulose, starch, calcium phosphate, etc.), binders (e.g., starch, sodium carmellose, hydroxypropyl cellulose, etc.), disintegrants (calcium carmellose, calcium carbonate, etc.), lubricants (magnesium stearate, talc, etc.), and so on, into tablets, capsules, granules, dry syrups, etc. that are commonly used for medical purposes. Further, since the crystalline compound according to the present invention is highly soluble in water and the like, it can be conveniently used for the production of and the preparation, when needed of various pharmacectical solutions including syrups. Moreover, a variety of these preparations may also be produced as sustained-release preparations that act for a prolonged period of time.

The crystalline compound of Compound A according to the present invention can be applied to various treatments in which administration routes other than oral administration are adopted. Non-limitative examples of formulations for use in such treatments include sublingual tablets, suppositories, inhalants, collunaria, eye drops, and percutaneously absorptive preparations such as plasters, ointments, and creams.

The crystalline compound of Compound A according to the present invention can be preferably produced in the following production process.
Compound A is dissolved in at least one organic solvent selected from N,N-dimethylformamide, dimethyl sulfoxide, and acetic acid, at a temperature between 20°C and 80°C. N,N-Dimethylformamide or dimethyl sulfoxide is herein preferred as the organic solvent. This solution is filtered, if desired, and is then added dropwise to stirred water at 20 to 40°C over approximately 1 to 3 hours, and the precipitate is collected by filtration. The precipitate is washed with water at 20 to 40°C and is dried at 20 to 60°C under reduced pressure to give the crystalline compound of Compound A according to the present invention.

As can be clearly known from the above, the present invention also provides, in another aspect, use of the crystalline compound of Compound A according to the present invention for the production of a pharmaceutical composition. Further, the present invention provides, in a further aspect, use of the crystalline compound of Compound A according to the present invention for the production of an antiallergic medicine. Furthermore, the present invention provides, in a still further aspect, a method for preventing or treating an allergic disease, comprising administering the crystalline compound of Compound A according to the present invention to an animal including a human.

### EXAMPLES

The present invention will now be explained more specifically by referring to the following Examples. However, these Examples are not intended to restrict the scope of the invention in any way. In the following Examples, the crystalline compound of Compound A according to the present invention is referred to as the "β-type crystalline material".

### Example 1: Process 1 for producing the β-type crystalline material of Compound A

25.0 g of the light yellow powder obtained in accordance with the process described in Example 20 of WO99/16770 was added to 0.53 liters of N,N-dimethylformamide and was dissolved therein with heating to approximately 50°C. The solution was filtered. The filtrate was added dropwise to 2.5 liters of stirred water over approximately one hour, and the precipitate was collected by filtration. The precipitate was washed twice with 1.25 liters of water, and was dried under reduced pressure at room temperature for 16 hours and then at 40°C for 1 day to give the β-type crystalline material (24.2 g, yield: 96.8%).

### Example 2: Process 2 for producing the β-type crystalline material of Compound A

25.6 g of the light yellow powder obtained in accordance with the process described in Example 20 of WO99/16770 was added to 1 liter of dimethyl sulfoxide and was dissolved therein with heating to approximately 60°C. The solution was filtered. The filtrate was added dropwise to 15 liters of water over approximately 1.5 hours, while stirring the water at a number of paddle revolutions of 200 rpm, and the precipitate was collected by filtration. The precipitate was washed twice with 2 liters of water, and was dried under reduced pressure at room temperature for 1 day and then at 40°C for 1 day to give the β-type crystalline material (23.5 g, yield: 91.8%).

### Example 3: Process 3 for producing the β-type crystalline material of Compound A

9.98 g of the light yellow powder obtained in accordance with the process described in Example 20 of WO99/16770 was added to 0.4 liters of N,N-dimethylformamide and was dissolved therein with heating to approximately 60°C. The solution was filtered. The filtrate was added dropwise to 6 liters of water over approximately 40 minutes, while stirring the water at a number of paddle revolutions of 200 rpm, and the precipitate was collected by filtration. The precipitate was washed twice with 1.2 liters of water, and was then dried under reduced pressure at room temperature for 1 day to give the β-type crystalline material (9.62 g, yield: 96.4%).

### Comparative Example 1: Process for producing the α-type crystalline material of Compound A

0.2 liters of methylene chloride was added to 10.01 g of the light yellow powder obtained in accordance with the process described in Example 20 of WO99/16770, and the mixture was stirred to give a suspension. Thereafter, the solvent contained in this suspension was distilled off by the use of a rotary evaporator. To the residue was added 0.2 liters of 2-propanol, and the mixture was stirred for 3 hours to give a suspension. This suspension was filtered, and to the residue was added again 0.2 liters of 2-propanol. The mixture was stirred for 3 hours to give a suspension. The suspension was filtered, and to the residue was added 0.2 liters of a 10% aqueous 2-propanol solution. The mixture was stirred for 1 day to give a suspension. This suspension was filtered, and the residue was dried under reduced pressure at room temperature for approximately 18 hours to give the α-type crystalline material of Compound A (9.79 g, yield: 97.8%).

### Test 1: Powder X-ray diffraction

The β-type and the α-type crystalline materials obtained in Example 1 and in Comparative Example 1, respectively, were identified by the use of a powder X-ray diffractometer. The measurement conditions were as follows:
Equipment: RINT 2100 (manufactured by Rigaku Corp., Japan)
Measurement conditions:
   X-ray: CuKα₁, tube voltage: 40 kV, tube electric current: 20 mA,
   Monochrome: graphite monochromator,
   scanning speed: 4°/min, scanning step: 0.02°,
   axis of scanning: 2θ/θ, scanning range: 2θ = 3 - 40°

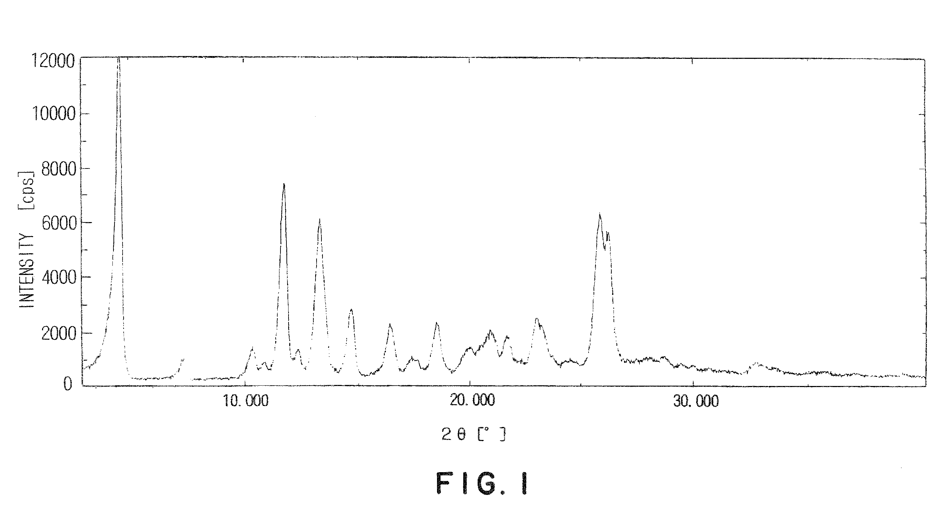

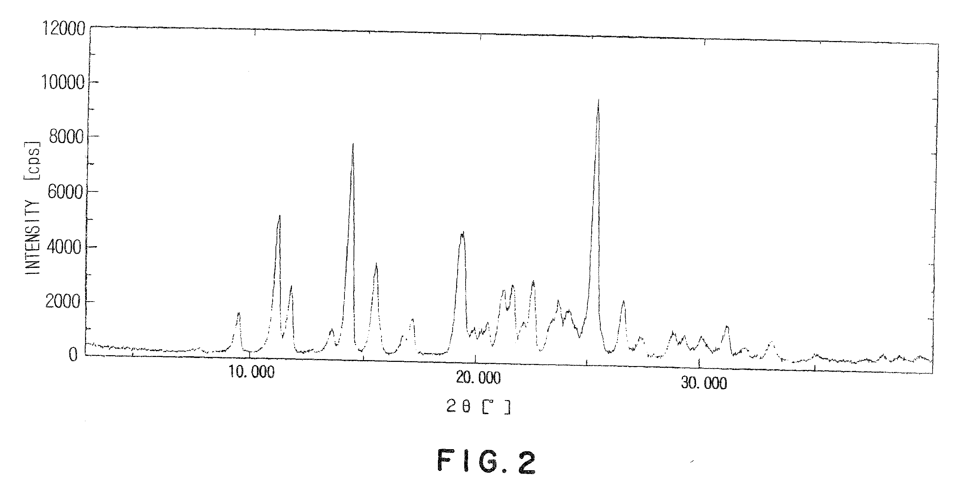

The powder X-ray diffraction pattern of the β-type crystalline material obtained in Example 1 was as shown in Fig. 1, and that of the α-type crystalline material obtained in Comparative Example 1 was as shown in Fig. 2. The β-type crystalline material had characteristic diffraction peaks at the diffraction angles (2θ) of: 4.7 ± 0.1°, 7.4 ± 0.1°, 11.8 ± 0.1°, 13.4 ± 0.1°, 16.5 ± 0.1°, and 18.6 ± 0.1°. On the other hand, the α-type crystalline material had characteristic diffraction peaks at the diffraction angles (2θ) of: 11.2 ± 0.1°, 14.4 ± 0.1°, 15.5 ± 0.1°, and 25.3 ± 0.1°. The powder X-ray diffraction pattern of the β-type crystalline material and that of the α-type crystalline material were found to be obviously different from each other.

### Test 2: DSC

The β-type and the α-type crystalline materials obtained in Example 1 and in Comparative Example 1, respectively, were analyzed by the use of a differential scanning calorimetry. The measurement conditions were as follows:
Equipment: DSC 220U (manufactured by Seiko Instruments, Inc., Japan)
Measurement conditions:
   pan: open aluminium pan, atmosphere: nitrogen,
   gas flow rate: 50 mL /min, heating rate: 5°C/min, measuring temperature range: 50 - 280°C.

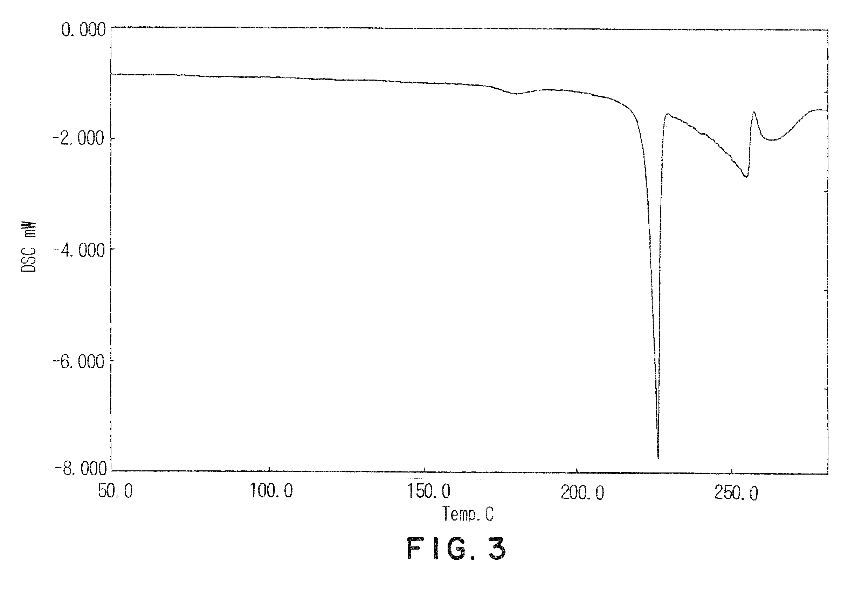

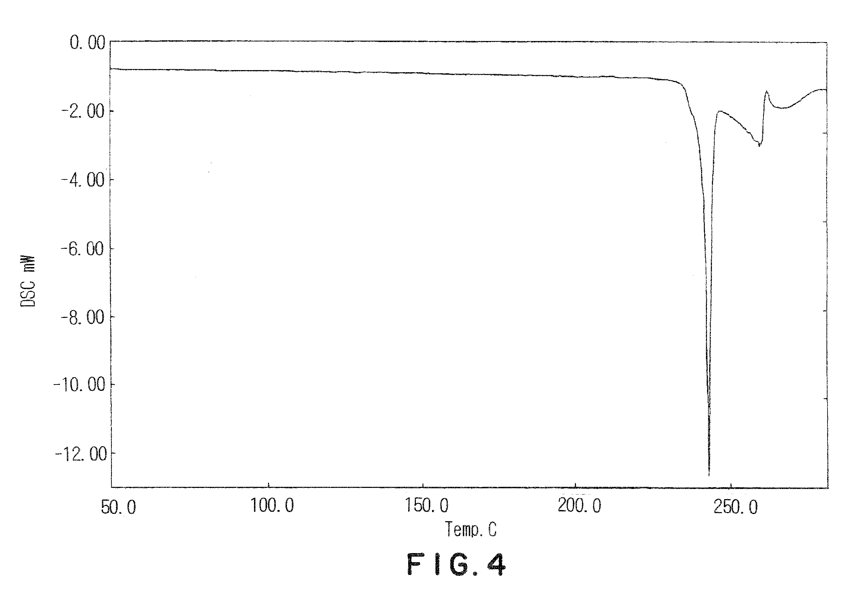

The β-type crystalline material obtained in Example 1 had, in the DSC curve shown in Fig. 3, a broad endothermic peak approximately at 170 - 190°C and a sharp endothermic peak approximately at 225°C. On the other hand, the α-type crystalline material obtained in Comparative Example 1 had, in the DSC curve shown in Fig. 4, an endothermic peak approximately at 243°C but no endothermic peaks approximately at 170 - 190°C and 225°C.

### Test 3: Solubility test

By using, as test samples, the β-type crystalline material obtained in Example 1 and the α-type crystalline material obtained in Comparative Example 1, a water (37°C) solubility test was carried out. The test sample (about 10 mg) was added to 100 mL of water (37°C), and the mixture was stirred at 1000 rpm. The mixture was sampled at some points of time, and each sample taken out was filtered through a membrane filter (Millex LG-13 manufactured by Millipore Corp, Japan). The compound A concentration of each filtrate was determined with high-performance liquid chromatography (HPLC). The measurement conditions were as follows:

HPLC: LC-10vp series (manufactured by Shimadzu Corp. Japan)
System controller: CBM-10A
Pump: LC-10Advp
Degasser: DGS-14A
Autosampler: SIL-10Advp
Column oven: CTO-ACvp
Detector: SPD-10Avp
Measurement wavelength: 246 nm
Column: Mightysil RP-18 GP 4.6 x 250 mm (manufactured by Kanto Chemical Co., Inc., Japan)
Column temperature: Fixed temperature at around 40°C
Mobile phase: Mixture of methanol and water (55:45)
Flow rate: 1 mL/min
Amount of influent: 10 µL
Internal standard solution: Acetonitrile solution of propyl paraoxybenzoate (conc.200 µg/mL)

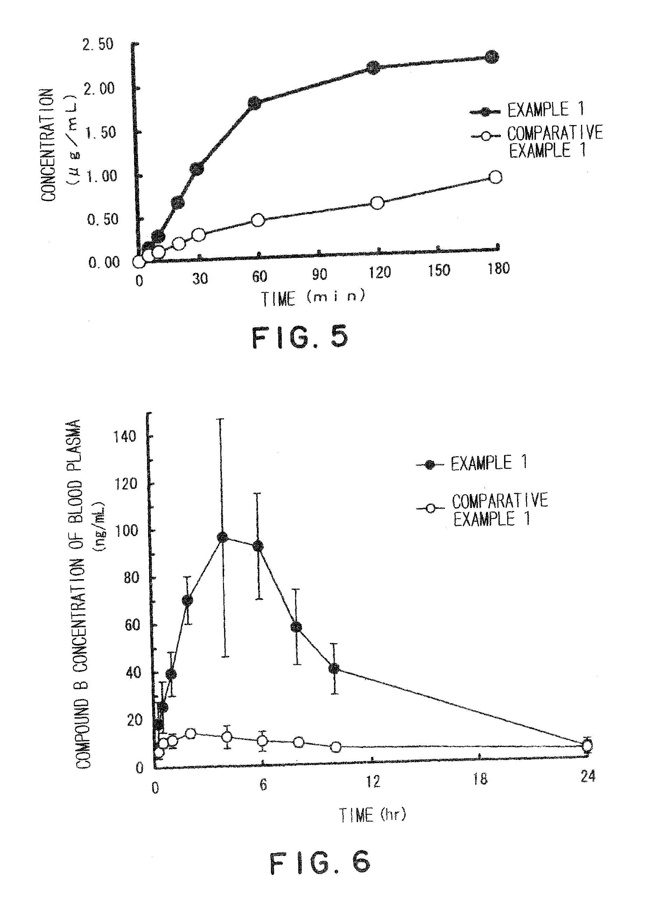

A change in the Compound A concentration of the sample solution of the β-type crystalline material obtained in Example 1 and a change in the Compound A concentration of the sample solution of the α-type crystalline material obtained in Comparative Example 1 were as shown in Fig. 5. The Compound A concentrations at each sampling time were compared, and it was found that the Compound A concentrations brought about by the β-type crystalline material were approximately 2 to 4 times higher than those brought about by the α-type crystalline material. This result demonstrates that the water solubility of the β-type crystalline material is higher than that of the α-type crystalline material.

### Test 4: Absorbability test

When absorbed into organisms, Compound A is converted into 7,8-dimethoxy-4(5H),10-dioxo-2H-1,2,3-triazolo[4,5-c][1] benzazepine (hereinafter referred to as Compound B), a main body that exhibits the physiological activity of Compound A. The following test was carried out, using Compound B as an indicator.
The α-type crystalline material obtained in Comparative Example 1 or the β-type crystalline material obtained in Example 1 was suspended in a 1 wt.% aqueous solution of methyl cellulose. Each suspension was orally administered to a group of cynomolgus monkeys that had not been fed one overnight (5 mg/kg, n=5). The two samples were compared, in terms of the change of Compound B concentration of blood plasma and the area under the medicine concentration in blood plasma-time curve (AUC), to evaluate the difference in absorbability between the samples. The Compound B concentration of blood plasma derived from the blood sample was determined in the following manner.

By centrifugally separating the blood (1 mL) taken from a saphenous vein (4°C, 3000 rpm, 10 minutes) in the presence of heparin, blood plasma was obtained. To this blood plasma (100 µL), a methanol solution containing an internal standard substance (sodium 7-methyl-4(5H),10-dioxo-2H-1,2,3-triazolo [4,5-c][1]benzazepine) (100 ng/mL, 100 µL) and methanol (400 µL) were added, and the mixture was stirred and was then subjected to centrifugal separation (4°C, 10000 rpm, 5 minutes). Methanol (300 µL) was added again to the supernatant, and the mixture was centrifugally separated under the same conditions as the previous manner. The supernatant was centrifuged under reduced pressure and was then evaporated to dryness. An HPLC mobile phase (150 µL) was added to the residue for redissolution, and the solution obtained was used as a sample for HPLC. The high-performance liquid chromatographic analysis conditions used in Test 4 were as follows:

HPLC pump: 600E (Nippon Waters Corp., Japan)
Autosampler: 717 plus (Nippon Waters Corp., Japan)
Detector: RF-10AXL (Shimadzu Corp., Japan)
Fluorescence detection wavelengths: Ex 270 nm, Em 466 nm
Column: Cosmosil 5C18-AR-II (4.6 x 150 mm, manufactured by NACALAI TESQUE, INC., Japan)
Guard column: Cosmosil 5C18-AR (4.6 x 10 mm, manufactured by NACALAI TESQUE, INC., Japan)
Column temperature: 35°C
Mobile phase: 10 mmol/liter phosphoric acid buffer (pH 7.0) : methanol (75:25)
Flow rate: 0.8 mL/min
Amount of influent: 20 µL

The results were as shown in Fig. 6. With respect to the Compound B concentration of blood plasma, the β-type-crystalline-material-administered group showed higher values than did the α-type-crystalline-material-administered group. Moreover, the AUC obtained from the β-type-crystalline-material-administered group was about 5 to 6 times greater than that obtained from the α-type-crystalline-material-administered group.

## Claims

1. Crystalline compound of 2-(1-isopropoxycarbonyloxy-2-methylpropyl)-7,8-dimethoxy-4(5H),10-dioxo-2H-1,2,3-triazolo[ 4,5-c][1]benzazepine, which has diffraction peaks at the diffraction angles (2θ) of: 4.7 ± 0.1°, 7.4 ± 0.1°, 11.8 ± 0.1°, 13.4 ± 0.1°, 16.5 ± 0.1°, and 18.6 ± 0.1°in a powder X-ray diffraction pattern.

2. The crystalline compound according to claim 1, which has endothermic peaks approximately at 170 ― 190°C and 225°C in a DSC chart obtained in differential scanning calorimetry (DSC).

3. A composition comprising the crystalline compound according to claim 1 or 2.

4. A pharmaceutical composition comprising the crystalline compound according to claim 1 or 2.

5. An antiallergic medicine comprising the crystalline compound according to claim 1 or 2.

6. Use of the crystalline compound according to claim 1 or 2 for the production of a pharmaceutical composition.

7. Use of the crystalline compound according to claim 1 or 2 for the production of an antiallergic medicine.

8. A method for preventing or treating an allergic disease, comprising administering the crystalline compound according to claim 1 or 2 to an animal including a human.
